(19) Europäisches Patentamt
European Patent Office
Office européen des brevets

(11) Veröffentlichungsnummer: **0 002 027**
**B1**

(12) # EUROPÄISCHE PATENTSCHRIFT

(21) Anmeldenummer: **78101327.1**

(22) Anmeldetag: **08.11.78**

(51) Int. Cl.³: **C 07 B 29/00,** //
C 07 C 69/145,
C 07 C 67/28,
C 07 C 33/02, C 07 C 29/00

(54) **Verfahren zur Herstellung von 2-Buten-1-ol-Verbindungen durch Isomerisierung der entsprechenden 3-Buten-1-ol-Verbindungen**

(30) Priorität: **19.11.77 DE 2751766**

(43) Veröffentlichungstag der Anmeldung:
**30.05.79 Patentblatt 79/11**

(45) Bekanntmachung des Hinweises auf die Patenterteilung:
**23.07.80 Patentblatt 80/15**

(84) Benannte Vertragsstaaten:
**BE CH DE FR GB NL**

(56) Entgegenhaltungen:
**DE - A - 1 901 709**

(73) Patentinhaber: **BASF Aktiengesellschaft**
**Carl-Bosch-Strasse 38**
**D - 6700 Ludwigshafen (DE)**

(72) Erfinder: **Rebafka, Walter, Dr.**
**Schuetzenstrasse 4**
**D - 6901 Eppelheim (DE)**

**EP 0 002 027 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 2-Buten-1-ol-Verbindungen durch Isomerisierung der entsprechenden 3-Buten-1-ol-Verbindungen

Die Erfindung betrifft ein verbessertes Verfahren zur Herstellung von 2-Buten-1-ol-Verbindungen durch Isomerisierung von 3-Buten-1-ol-Verbindungen in Gegenwart von Palladium-Katalysatoren, die mit Selen oder Tellur dotiert sind, und Wasserstoff.

Es ist aus Tetrahedron, Band 20 (1964), Seiten 2911 f. bekannt, daß Allycarbinol in Gegenwart von N-Lithium-äthylendiamin als Katalysator teilweise zu Crotylalkohol isomerisiert. Weiterhin ist es bekannt, ungesättigte Alkohole in Gegenwart von Carbonylen der Metalleder 8. Gruppe des periodischen Systems als Katalysatoren zu isomerisieren. Dieses Verfahren hat jedoch den Nachteil, daß man bei der Isomerisierung zahlreiche Neben- und Folgeprodukte, z.B. entsprechende Aldehyde, erhält (vgl. Chem. Comm., 1968, Seiten 97—99 und J. of the Am. Chem. Soc., Band 85 (1963) Seite 1549).

Man kann die Isomerisierung auch ohne Verwendung von Katalysatoren rein thermisch durchführen (vgl. Can. Journ. of Chem., Band 46 (1968) Seite 2225), man muß jedoch zumeist sehr hohe Temperaturen anwenden, die zur teilweisen Verharzung der Ausgangsverbindung führen.

In der deutschen Patentschrift 1 901 709 wird ein Verfahren beschrieben, bei dem die Isomerisierung einer Doppelbindung unter dem katalytischen Einfluß von Palladium und oder seinen Verbindungen und Wasserstoff durchgeführt wird.

Obwohl das letztgenannte Verfahren gegenüber den vorgangehend aufgeführten Isomerisierungsmethoden sowohl in der technischen Durchführung als auch hinsichtlich der dabei erzielten Ausbeuten beträchtliche Vorteile bietet, arbeitet es dennoch nicht optimal:

Bei der Isomerisierung von Doppelbindungen mit Palladium und Wasserstoff entsteht durch Hydrierung außer dem gewünschten doppelbindungsisomeren Produkt auch die entsprechende gesättigte Verbindung in mehr oder minder großem Maße.

Obwohl die Minimierung der Hydrierreaktioren schon allein vom Standpunkt der Optimierung der Syntheseausbeute wünschenswert ist, ergibt sich noch aus einem anderen Grund ein Zwang zur Reduzierung des Anfalls on Hydrierprodukten:

Bei der Doppelbindungsisomerisierung von substituierten Butenolen werden keine vollständigen Stoffumsätze erhalten, sondern es stellt sich ein Gleichgewicht der Doppelbindungsisomeren ein, das den relativen thermodynamischen Stabilitäten der Produkte entspricht.

Um das Isomerisierungsverfahren wirtschaftlich zu gestalten, ist es daher nötig, das unumgesetzte Ausgangsprodukt nach destillativer Abtrennung vom Reaktionsprodukt wieder in die Synthese zurückzuführen.

Bei manchen Butenolen ist aufgrund zu geringer Siedepunkt-differenz zwischen unumgestztem Ausgangsprodukt und Hydrierprodukt eine destillative Ausschleusung des unerwünschten Nebenprodukts nicht oder nur mit unverhältnismäßig großem Aufwand möglich. Z.B. beträgt der Siedepunkt von 3-Methyl-3-buten-1-ol 131,5°C bei 1 020 mbar und der des entsprechenden Hydrierungsproduktes (3-Methyl-1-butenol) 130,9°C; oder der Siedepunkt von 3-Methyl-3-buten-1-yl-acetat beträgt 142,5°C bei 1 020 mbar, der des entsprechenden Hydrierungsproduktes (Isoamylacetat) 144°C.

Eine Differenzierung zwischen unumgesetztem Ausgansprodukt und dem Hydrierprodukt ist durch fraktionierte Destillation nicht möglich, sondern bedarf feinerer Anaylsenmethoden.

Aufgrund der nur destillativen Aufarbeitung der Reaktionsprodukte in den Beispielen der DE 19 01 709 wurde daher der Anteil an Hydrierprodukt nicht erkannt.

Es zeigte sich, daß bei Verwendung geeigneter Anaylsenmethoden die Anwendung des Verfahrens nach DE 19 01 709 merkliche Mengen an Hydrierungsprodukten mit sich bringt.

Um bei der Rückführung des Einsatzmaterials eine Anreicherung des Hydrierprodukts zu verhindern, ist es in diesen Fällen nötig, das Hydrierprodukt unter Wertproduktverlusten als Teilstrom auszuschleusen.

Die Wertproduktverluste werden bei diesem Vorgehen umso kleiner, je geringer der Anfall von Hydrierprodukt pro Synthesecyclus ist.

Eine Minimierung der Hydrierreaktion ist daher für die Wirtschaftlichkeit des Isomerisierungsverfahrens zwingend gegeben.

Es wurde nun überraschenderweise gefunden, daß man bei der isomerisierung von ungesättigten Verbindungen in Gegenwart von Palladium und/oder seinen Verbindungen und Wasserstoff die Bildung der Hydriernebenprodukte beträchtlich vermindern kann, wenn man die Isomerisierung in Gegenwart von Selen und/oder Tellur und/oder deren Verbindungen als Cokatalysator durchführt.

Gegenstand der Erfindung ist dementsprechend ein Verfahren zur Herstellung von 2-Buten-1-ol-Verbindungen der allgemeinen Formel I

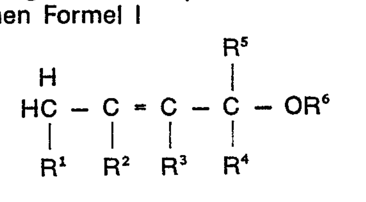

(I),

in der die einzelnen Reste $R^1$, $R^2$, $R^3$, $R^4$, $R^5$ und $R^6$ gleich oder verschieden sein können und jeweils ein Wasserstoffatom oder einen Aklylrest bedeuten, darüber hinaus $R^2$ auch den Rest-CHO bezeichnen kann, $R^2$ und $R^5$ zusammen mit den zwischen ihnen liegenden Kohlenstoffatomen auch Glieder eines alicyclischen Ringes bedeuten können, und/oder $R^6$ auch für einen cycloaliphatischen, araliphatischen, aromatischen Rest oder den Rest

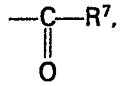

in dem $R^7$ einen aliphatischen, cycloaliphatischen, araliphatischen oder aromatischen Rest bedeutet, steht, durch isomerisierung von 3-Buten-1-ol-Verbindungen der allgemeinen Formel II

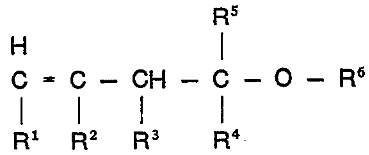

(II),

in der $R^1$, $R^2$, $R^3$, $R^4$, $R^5$ und $R^6$ die vorgenannte Bedeutung haben in Gegenwart von Palladium und/oder seinen Verbindungen und Wasserstoff bei Temperaturen von 0 bis 250°C dadurch gekennzeichnet, daß man die Isomerisierung in Gegenwart von Selen und/oder Tellur und/oder deren Verbindungen als Cokatalysator vornimmt.

Die Umsetzung läßt sich für den Fall der Verwendung von 3-Methyl-3-buten-1-ol durch folgende Reaktionsgleichung wiedergeben:

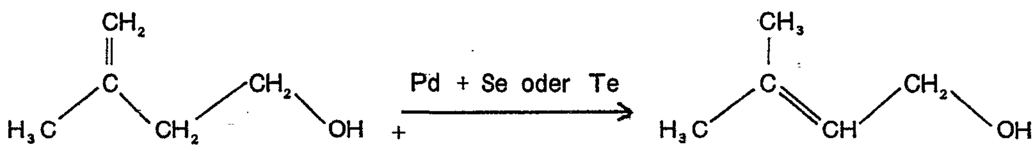


Im Vergleich zu den genannten bekannten Verfahren liefert das Verfahren nach der Erfindung die gewünschten 2-Buten-1-ol-Verbindungen auf wirtschaftlichem Wege, in besserer Ausbeute und Reinheit bei einem geringeren Anfall von Hydrierprodukten als nach den bekannten Verfahren.

Als Ausgangsstoffe verwendet man 3-Buten-1-ole der allgemeinen Formel II bzw. seine Ester oder Ather. Bevorzugte Ausganssttoffe II und dementsprechend auch bevorzugte Endstoffe I sind solche, in deren Formeln die einzelnen Reste $R^1$, $R^2$, $R^3$, $R^4$, $R^5$ und $R^6$ gleich oder verschieden sein können und jeweils ein Wasserstoffatom oder einen Alkylrest mit 1 bis 6 Kohlenstoffatomen, vorzugsweise ein Wasserstoffatom oder Methyl, bedeuten, sowie Verbindungen, in denen $R^2$ den Rest —CHO bedeutet; in denen $R^2$ und $R^5$ zusammen mit den zwischen ihnen liegenden Kohlenstoffatomen Glieder eines 5-, 6- oder 7-gliedrigen, alicyclischen Ringes bedeuten, Verbindungen, in denen $R^6$ für einen Cycloalkylrest mit 5 bis 7 Kohlenstoffatomen, einen Aralkylrest mit 7 bis 12 Kohlenstoffatomen, einen Phenylrest, Naphthylrest oder den Rest

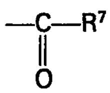

steht, in dem $R^7$ einen Alkylrest mit 1 bis 6 Kohlenstoffatomen, einen Cycloalkylrest mit 5 bis 7 Kohlenstoffatomen, einen Aralkylrest mit 7 bis 12 Kohlenstoffatomen, einen Phenylrest oder Naphthylrest bedeutet. Die genannten Reste können auch durch unter den Reaktionsbedingungen inerte Gruppen und/oder Atome, z.B. Äthergruppen, substituiert sein.

Als Ausgangsstoffe der allgemeinen Formel II seien beispielsweise genannt: 3-Buten-1-ol; 3-Methyl-3-buten-1-ol; 3-Formyl-3-buten-1-ol; 3,2,1-Trimethyl-3-buten-1-ol; 2-Isobutyl-3-buten-1-ol; 1-Hexyl-3-buten-1-ol; 1-Methylen-2-methyl-cyclohexan-3-ol; 1-Methylen-2-äthyl-cyclopentan-3-ol; 1-Methylen-cyclohexan-3-ol; 1-Methylen-cycloheptan-3-ol sowie die entsprechenden Äthyl-, Cyclohexyl-, Benzyl-, Phenyl- und $\alpha$-Naphthyläther bzw. die entsprechenden Essigsäure-, Cyclohexancarbonsäure-, Benzoesäure-, $\alpha$-Naphthoesäure- oder Dihydrozimtsäure-ester.

Besonderen Vorteil bringt das erfindungsgemäße Verfahren bei der Isomerisierung von 3-Methyl-3-buten-1-ol bzw. seiner am Sauerstoff substituierten Derivate (Acetate, Äther) zu Prenol oder den entsprechenden Prenylderivaten.

Die Umsetzung wird in Gegenwart von Palladium und/oder seinen Verbindungen durchgeführt. Als Isomerisierungskatalysatoren können demnach z.B. Palladiummohr, Palladiumpulver, Palladiumbromid, -arsenid, -cyanid, -chlorid,- nitrat, -jodid, -oxid, -sulfid, -sulfat oder auch Palladium-Komplexsalze wie Tetrachlorpalladate, verwendet werden. Vorteilhaft werden die genannten Katalysatoren auch in bekannter Weise auf Träger, z.B. Aktivkohle, Bariumsulfat, Kieselgel, Aluminiumoxid oder Zeolithe aufgebracht und die erhaltenen Trägerkatalysatoren für die Isomerisierung verwendet. Die Herstellung solcher Trägerkatalysatoren kann sich in beliebiger Weise, z.B. durch Tränken des Trägers mit entsprechenden Lösungen der Palladiumsalze, durch Verkneten bzw. Mischen unter Vermahlen der Komponenten, vollziehen. Bezüglich Details der Herstellung von Katalysatoren, insbesondere Trägerkatalysatoren, wird auf Houben-Weyl, Methoden der Organischen Chemie, Band 4/2, Seiten 137 ff. verwiesen. Den Palladiumkatalysator verwendet man im allgemeinen in einer Menge von 0,01 bis 5 Gew.%, vorzugsweise 0,1 bis 2 Gew.% Palladium, bezogen auf Ausgangsstoff II, in Form von feinverteiltem Metall oder in einer Menge von 0,01 bis 5 Gew.%, vorzugsweise 0,1 bis 2 Gew.%, berechnet als Palladium, bezogen auf Ausgangsstoff II, in Form feinverteilter Pd-Verbindungen.

Als erfindungsgemäße, vorteilhafte Cokatalysatoren werden Selen und/oder Tellur als Element oder deren Verbindungen, wie Selendioxid, selenige Säure, Selensäure, Tellurdioxid, Tellurtrioxid, tellurige Säure oder o-Tellursäure verwendet.

Die Art der eingesetzten Se- oder Tellur-Verbindung ist nicht kritisch, da unter den Reaktionsbedingungen (Anwesenheit von $H_2$/Pd) die Selen- oder Tellurverbindungen mit einer Oxidationsstufe großer als 0 in den nullwertigen Zustand überführt werden. Der oder die Zusatzstoff(e) wird (werden) in einer Menge von 1 bis 100 Gew.%, vorzugsweise 2 bis 10 Gew.% bezogen auf das metallische Palladium, eingesetzt.

Die Art und Weise der Zugabe des Cokatalysators zum Katalysator oder zum Reaktionsgemisch ist nicht kritisch. So kann man beispielsweise Pd-Verbindungen und Se- oder Te-Verbindungen gemeinsam im zu isomerisierenden Alkohol lösen und mit $H_2$ gemeinsam fällen; oder aber man kann beide Komponenten gemeinsam auf einen Träger auftragen und den so erhaltenen Trägerkatalysator nach üblicher Behandlung in die Synthese einzetzen; oder man kann auf einen Pd-Trägerkatalysator nachträglich Te und/oder Se und/oder deren Verbindungen auftragen; oder aber den Cokatalysator als lösliche Verbindung im zu isomerisierenden Aklohol lösen und in Laufe der Reaktion mit Hilfe des Wasserstoffs auf einen bereits vorgefertigten Pd-Trägerkatalysator niederschlagen.

Die Umsetzung wird in Gegenwart von Wasserstoff durchgeführt. Man kann den Wasserstoff kontinuierlich oder diskontinuierlich der Reaktion zuführen und/oder den Kontakt selbst nach einer bestimmten Reaktionszeit wieder frisch mit Wasserstoff beladen. Den Wasserstoff verwendet man im allgemeinen in einer Menge von 1 bis 50 Mol%, bezogen auf den Ausgangsstoff II.

Die Isomerisierung erfolgt in der Regel bei einer Temperatur zwischen 0 und 250°C, vorzugsweise zwischen 30 und 150°C, drucklos oder unter bis zu 50 bar erhöhtem Druck, kontinuierlich oder diskontinuierlich. Gegebenenfalls verwendet man unter den Reaktionsbedingungen inerte organische Lösungsmittel wie Äther, z.B. Diäthyläther, Dioxan, Tetrahydrofuran; Alkanole, z.B. Äthanol, Isobutanol; aromatische oder aliphatische Kohlenwasserstoffe, z.B. Heptan, Benzol; oder entsprechende Gemische. Man kann auch Lösungsmittel verwenden, die unter den Reaktionsbedingungen als Wasserstoffdonatoren dienen und dabei selbst dahydriert werden, wie Derivate des Cyclohexens, z.B. Cyclohexenol, Tetrahydroacetophenon.

Zur Durchführung der erfindungsgemäßen Umsetzung hält man im allgemeinen den Ausgangsstoff der Formel II, den Katalysator, den Cokatalysator und gegebenenfalls das inerte Lösungsmittel zusammen für etwa 15 bis 360 Minuten bei der Reaktionstemperatur.

Anschließend wird der Endstoff in üblicher Weise, z.B. durch fraktionierte Destillation, aus dem Gemisch abgetrennt.

Die nach dem Verfahren der Erfindung herstellbaren Verbindungen sind zum Teil Lösungsmittel, zum anderen Teil wertvolle Ausgangsstoffe für die Herstellung von Lösungsmitteln, Farbstoffen, Lacken, Anstrichmitteln und Schädlingsbekämpfungsmitteln.

In den folgenden Beispielen wird anhand von Vergleichsversuchen mit und ohne erfindungsgemäßen Cokatalysator der Vorteil der Erfindung erläutert.

## Beispiel 1

300 g 3-Methyl-3-buten-1-yl-acetat (MBE-ac) wurden unter kräftigem Rühren und gleichzeitigem Einleiten von Wasserstoff 2 Stunden lang in Gegenwart von 0,6 g eines Katalysators, der 5 % Pd und 0,25 % Se auf Aktivkohle enthält, auf 85°C erhitzt, und im Reaktionsgemisch wurde in aus der folgenden Tabelle ersichtlichen Zeitabständen gaschromatographisch der Prozentgehalt (Gew.%) an MBE-ac als Indikator für den Fortgang der Umsetzung, der Prozentgehalt an 3-Methyl-butan-1-yl-acetat (MBA-ac) als Maß für die Bildung an unerwünschtem Hydrierungsprodukt sowie der Prozentgehalt an gewünschtem 3-Methyl-2-buten-1-yl-acetat (Prenylacetat) bestimmt.

Zum Vergleich wurde der Versuch wiederholt, jedoch unter Verwendung eines Katalysators, der 5 % Pd, aber kein Se auf Aktivkohle enthält.

Die Ergebnisse sind in der folgenden Tabelle dargestellt.

| Zeit [Min] | Isomerisierung an Pd/Se/C MBE–ac [% Gehalt] | MBA–ac [% Gehalt] | Prenylacetat [% Gehalt] | Isomerisierung an Pd/C (Vergleichsversuch) MBE–ac [% Gehalt] | MBA–ac [% Gehalt] | Prenylacetat [% Gehalt] |
|---|---|---|---|---|---|---|
| 15 | 42 | 4,5 | 51,1 | 49 | 11 | 37 |
| 30 | 25,5 | 8,5 | 62 | 30 | 17,5 | 50 |
| 45 | 22 | 12 | 63 | 27 | 27 | 54 |
| 60 | 21 | 14 | 61 | 25,5 | 31 | 50 |

Beispiel 2

300 g 3-Buten-1-ol wurden unter kräftigem Rühren und gleichzeitigem Einleiten von Wasserstoff 2 Stunden lang in Gegenwart von 0,6 g eines Katalysators, der 5 % Pd und 0,25 % Se auf Aktivkohle enthielt, auf 85°C erhitzt und analog Beispiel 1 wurde in Abhängigkeit von der Reaktionszeit der Umsatz, die Bildung an unerwüschtem Hydrierprodukt sowie die Bildung von cis-trans-2-Butenolen ermittelt.

Zum Vergleich wurde der Versuch wiederholt, jedoch unter Verwendung eines Katalysators, der 5 % Pd, aber kein Se auf Aktivkohle enthält.

Die Ergebnisse sind in der folgenden Tabelle dargestellt.

| Zeit [Min] | Isomerisierung an Pd/Se/C 3-Buten-1-ol [% Gehalt] | 1-Butanol [% Gehalt] | cis-trans-2-Butenole [% Gehalt] | (Vergleichsversuch) Isomerisierung an Pd/C 3-Buten-1-ol [% Gehalt] | 1-Butanol [% Gehalt] | cis-trans-2-Butenole [% Gehalt] |
|---|---|---|---|---|---|---|
| 30 | 64 | 4 | 29 | 71 | 6 | 21 |
| 60 | 44 | 7 | 42 | 49 | 12 | 36 |
| 90 | 28,5 | 10 | 57 | 32 | 15,5 | 49 |
| 120 | 27 | 12,5 | 65 | 27,5 | 19 | 59 |

Beispiel 3

500 g 3-Methyl-3-buten-1-ol (MBE) wurden unter kräftigem Rühren und gleichzeitigem Einleiten von Wasserstoff 2 Stunden lang in Gegenwart von 1 g eines Katalysators, der 5 Gew.% Pd und 0,25 % Se auf Aktivkohle enthielt, auf 85°C erhitzt und analog Beispiel 1 wurde in Abhängigkeit von der Reaktionszeit der Umsatz, die Bildung an unerwünschtem Hydrierungsprodukt 3-Methyl-butanol (MBA) sowie die Bildung von Prenol ermittelt.

Zum Vergleich wurde der Versuch wiederholt, jedoch unter Verwendung eines Katalysators, der 5 % Pd, aber kein Se auf Aktivkohle enthält.

Die Ergebnisse sind in der folgenden Tabelle dargestellt.

| Zeit [Min] | Isomerisierung an Pd/Se/c | | | Isomerisierung an Pd/C | | |
|---|---|---|---|---|---|---|
| | MBE [% Gehalt] | MBA [% Gehalt] | Prenol [% Gehalt] | MBE [% Gehalt] | MBA [% Gehalt] | Prenol [% Gehalt] |
| 30 | 52,5 | 1 | 45 | 44,5 | 4 | 50 |
| 60 | 40 | 1,5 | 57 | 35 | 6 | 57 |
| 90 | 32 | 2 | 64 | 31 | 7,5 | 57 |
| 120 | 30 | 2,3 | 62 | 30 | 10,5 | 56,5 |

Beispiel 4

500 Teile 3-Methyl-3-buten-1-ol wurden gemeinsam mit 2 Teilen eines Katalysators, der 4,3 % Pd, 0,9 % Te auf einem Aluminiumsilikat enthielt, 2 Stunden lang bei 110°C in Anwesenheit von Wasserstoff gerührt. Bei einem Reaktionsumsatz von 60 % 3-Methyl-3-buten-1-ol wurden nur 1,8 % 3-Methyl-butan-1-ol und 55,4 % Prenol gebildet.

Beispiel 5

500 Teile 3-Methyl-3-buten-1-ol wurden mit 1 Teil Katalysator, der aus 4,4 % Pd und 0,8 % Te auf Kohle bestand, 2 Stunden lang bei 110°C in Anwesenheit von Wasserstoff gerührt. Der Reaktionsaustrag enthielt bei einem 3-Methyl-3-buten-1-ol Umsatz von 48 % nur 1,7 % 3-Methyl-butan-1-ol und 46 % Prenol.

Beispiel 6

In eine Lösung von 2,5 mg $SeO_2$ in 3-Methyl-3-buten-1-ol (MBE) wurden 2 g eines Pd-Katalysators gegeben, der 5 % Pd auf Kohle enthielt. Unter Rühren wurde 1 Stunde lang bei Raumtemperatur und dann eine Stunde lang bei 110°C $H_2$ eingeleitet.

Bei einem MBE-Umsatz von 55 % wurden nur 2 % 3-Methyl-butanol und 52 % Prenol gebildet.

**Patentanspruch**

Verfahren zur Herstellung von 2-Buten-1-ol-Verbindungen der allgemeinen Formel I

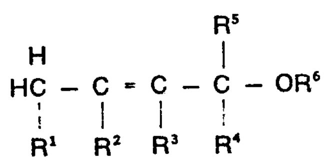

(I),

in der die einzelnen Reste $R^1$, $R^2$, $R^3$, $R^4$, $R^5$ und $R^6$ gleich oder verschieden sein können und jeweils ein Wasserstoffatom oder einen Alkylrest bedeuten, darüber hinaus $R^2$ auch den Rest —CHO bezeichnen kann, $R^2$ und $R^5$ zusammen mit den zwischen ihnen liegenden Kohlenstoffatomen auch Glieder eines alicylischen Ringes bedeuten können, und/oder $R^6$ auch für einen cycloaliphatischen, araliphatischen, aromatischen Rest oder den Rest

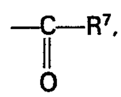

in dem $R^7$ einen aliphatischen, cycloaliphatischen, araliphatischen oder aromatischen Rest bedeutet, steht, durch Isomerisierung von 3-Buten-1-ol-Verbindungen der allgemeinen Formel II

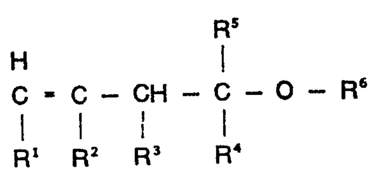

(II),

in der $R^1$, $R^2$, $R^3$, $R^4$, $R^5$ und $R^6$ die vorgenannte Bedeutung haben, in Gegenwart von Palladium und/oder seinen Verbindungen und Wasserstoff bei Temperaturen von 0 bis 250°C, *dadurch gekennzeichnet,* daß man die Isomerisierung in Gegenwart von Selen und/oder Tellur und/oder deren Verbindungen als Cokatalysator vornimmt.

**Claim**

Process for the production of 2-buten-1-ol compounds of the general formula I

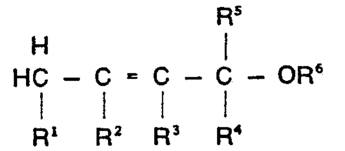

(I),

where the individual radicals $R^1$, $R^2$, $R^3$, $R^4$, $R^5$ and $R^6$ are identical or different and each denotes a hydrogen atom or an alkyl radical, $R^2$ may also denote the radical —CHO, $R^2$ and $R^5$ may also, together with the carbon atoms situated between them, denote members of an alicyclic ring, and/or $R^6$ may also denote a cycloaliphatic, araliphatic or aromatic radical or the radical

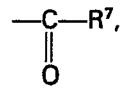

where $R^7$ denotes an aliphatic, cycloaliphatic, araliphatic or aromatic radical, by isomerization of 3-buten-1-ol compounds of the general formula II

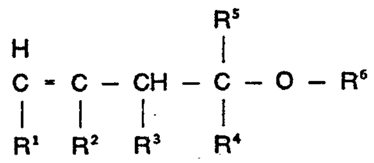

(II),

where $R^1$, $R^2$, $R^3$, $R^4$, $R^5$ and $R^6$ have the above meanings, in the presence of palladium and/or compounds thereof and hydrogen at temperatures of 0 to 250°C, *characterized in that* the isomerization is carried out in the presence of selenium and/or tellurium and/or compounds thereof as cocatalyst.

**Revendication**

Procédé de préparation de composés 2-buten-1-oliques de formule générale 1

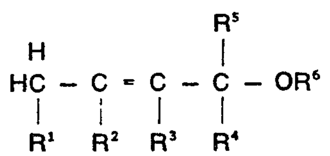

(I)

dans laquelle les différents radicaux $R^1$, $R^2$, $R^3$, $R^4$, $R^5$ et $R^6$ peuvent être semblables ou dissemblables et représentent chacun un atome d'hydrogène ou un radical alcoyle, $R^2$ pouvant en outre représenter le radical —CHO, $R^2$ et $R^5$ pouvant former, avec les atomes de carbone qui sont situés entre eux, des termes d'un noyau alicylique et/ou $R^6$ pouvant être également mis pour un radical cycloaliphatique, araliphatique, aromatique ou pour le radical

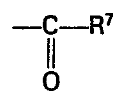

($R^7$ étant un radical aliphatique, cycloaliphatique, araliphatique ou aromatique), par isomérisation de composés 3-butèn-1-oliques de formule générale II

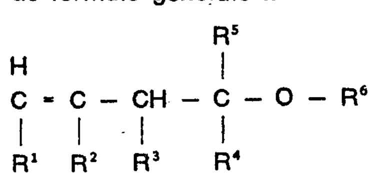

(II)

dans laquelle $R^1$, $R^2$, $R^3$, $R^4$, $R^5$ et $R^6$ ont les significations données ci-dessus, en présence de palladium et/ou de ses composés et d'hydrogène à une température comprise entre 0 et 250°C, caractérise en ce qu'on procède à l'isomérisation en présence de sélénium et/ou de tellure et/ou de leurs composés en tant que co-catalyseur.